(19) Europäisches Patentamt
European Patent Office
Office européen des brevets

(11) Veröffentlichungsnummer : **0 432 099 A2**

# (12) EUROPÄISCHE PATENTANMELDUNG

(21) Anmeldenummer : **90810933.3**

(22) Anmeldetag : **29.11.90**

(51) Int. Cl.[5] : **C07D 487/20**, C07D 471/20, A61K 31/495, A61K 31/435, A61K 31/415, // (C07D487/20, 241:00, 209:00, 209:00), (C07D487/20, 243:00, 209:00, 209:00), (C07D471/20, 241:00, 221:00, 209:00), (C07D471/20, 243:00, 221:00, 209:00)

(30) Priorität : **08.12.89 CH 4412/89**

(43) Veröffentlichungstag der Anmeldung : **12.06.91 Patentblatt 91/24**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Fröstl, Wolfgang, Dr.**
**St. Johanns-Vorstadt 6/2**
**CH-4056 Basel (CH)**
Erfinder : **Mondadori, Cesare, Dr.**
**Traugott Meyer-Strasse 70**
**CH-4147 Aesch (CH)**
Erfinder : **Strub, Dietrich**
**Lavaterstrasse 14**
**CH-4127 Birsfelden (CH)**
Erfinder : **Züst, Armin, Dr.**
**Oetlingerstrasse 194/4**
**CH-4057 Basel (CH)**

## (54) Hydrierte Stickstoffheterocyclen.

(57) Neue hydrierte, zwei Stickstoffatome enthaltende, heterocyclische Verbindungen der Formel

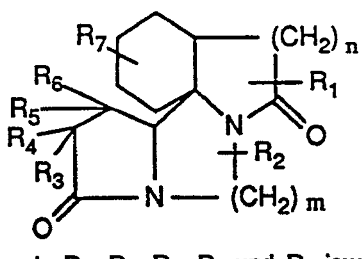

(I),

worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ entweder jeweils Wasserstoff bedeuten oder gemeinsam für eine zusätzliche Bindung stehen, in freier Form oder in Salzform, können als pharmazeutische Wirkstoffe verwendet werden und sind in an sich bekannter Weise herstellbar.

EP 0 432 099 A2

# HYDRIERTE STICKSTOFFHETEROCYCLEN

Die Erfindung betrifft hydrierte, zwei Stickstoffatome enthaltende, heterocyclische Verbindungen der Formel

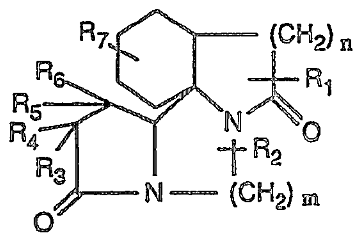

(I),

worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ entweder jeweils Wasserstoff bedeuten oder gemeinsam für eine zusätzliche Bindung stehen, in freier Form oder in Salzform, die Verwendung dieser Verbindungen, ein Verfahren zur Herstellung dieser Verbindungen und pharmazeutische Zusammensetzungen, enthaltend eine solche Verbindung I in freier Form oder in Form eines pharmazeutisch verwendbaren Salzes.

Die Verbindungen I können im Rahmen der Erfindung in Form von Stereoisomeren vorliegen. Da die Verbindungen I mindestens drei chirale Kohlenstoffatome (C-Atome) besitzen (nämlich die C-Atome, die an der Verknüpfung der vier (theoretischen) isolierten Ring-Grundstrukturen zu dem in der Formel I gezeigten tetracyclischen Gerüst beteiligt sind), können sie z.B. als reine Enantiomere, Enantiomerengemische, wie Racemate, reine Diastereomere, Diastereomerengemische oder Racematgemische vorliegen. Bevorzugt sind im Rahmen der Erfindung Verbindungen I, welche an den drei vorstehend erwähnten chiralen C-Atomen die beispielsgemäss offenbarte Stereochemie aufweisen.

Verbindungen I in Salzform sind insbesondere entsprechende Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet. Umfasst sind ferner für pharmazeutische Verwendungen ungeeignete Salze, da diese beispielsweise für die Isolierung bzw. Reinigung freier Verbindungen I sowie deren pharmazeutisch verwendbarer Salze verwendet werden können.

Vor- und nachstehend sind unter mit "Nieder" bezeichneten Resten oder Verbindungen, sofern nicht abweichend definiert, solche zu verstehen, die bis und mit 7, vor allem bis und mit 4, Kohlenstoffatome enthalten.

Niederalkyl ist $C_1$-$C_4$-Alkyl, d.h. Methyl, Aethyl, n-Propyl, Isopropyl, n-Butyl, Isobutyl, sek-Butyl oder tert-Butyl, und umfasst ferner $C_5$-$C_7$-Alkyl-, d.h. entsprechende Pentyl-, Hexyl- oder Heptylreste.

Halogen ist Halogen mit einer Atomnummer bis und mit 53, d.h. Chlor oder Brom, ferner Fluor oder Iod.

Die Verbindungen I weisen beispielsweise wertvolle pharmakologische, insbesondere nootrope, Eigenschaften auf.

So bewirken sie z.B. im Two-Compartment Passive Avoidance-Testmodell [nach Mondadori und Classen, Acta Neurol. Scand. 69, Suppl. 99, 125 (1984)] in Dosismengen ab etwa 0,3 mg/kg i.p. sowie p.o. bei der Maus eine deutliche Reduktion der amnesischen Wirkung eines zerebralen Elektroschocks.

Ebenso besitzen die Verbindungen I eine beträchtliche gedächtnisverbessernde Wirkung, die im Step-down Passive Avoidance-Test [nach Mondadori und Waser, Psychopharmacol. 63, 297 (1979)] ab einer Dosis von etwa 0,3 mg/kg p.o. an der Maus festzustellen ist.

Weiterhin zeigen die Verbindungen I eine signifikante gedächtnisauffrischende Aktivität, die anhand des Step-through Dark Avoidance-Testmodells ab einer Dosis von etwa 0,3 mg/kg p.o. an der Maus verifiziert werden kann.

Entsprechend können die Verbindungen I, in freier Form oder in pharmazeutisch verwendbarer Salzform, z.B. als Wirkstoffe in Nootropika verwendet werden, welche z.B zur Behandlung von cerebralen Insuffizienzerscheinungen, insbesondere Gedächtnisstörungen, eingesetzt werden. Ein Gegenstand der Erfindung ist somit die Verwendung der Verbindungen I, in freier Form oder in pharmazeutisch verwendbarer Salzform, zur Herstellung von entsprechenden Arzneimitteln. Dabei ist die gewerbsmässige Herrichtung der Wirksubstanzen eingeschlossen.

Bevorzugt sind im Rahmen der Erfindung Verbindungen der Formel I, worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ jeweils Wasserstoff bedeuten, in freier Form oder in Salzform.

Besonders bevorzugt sind im Rahmen der Erfindung Verbindungen der Formel I, worin $R_1$, $R_2$, $R_3$, $R_5$ und $R_7$ jeweils unabhängig voneinander Wasserstoff oder Niederalkyl bedeuten, m für 2 steht, n für 1 steht und $R_4$ und $R_6$ entweder jeweils Wasserstoff bedeuten oder gemeinsam für eine zusätzliche Bindung stehen, in freier Form oder in Salzform.

In besonderer Weise bevorzugt sind im Rahmen der Erfindung Verbindungen der Formel I, worin $R_1$, $R_2$, $R_3$, $R_5$ und $R_7$ jeweils unabhängig voneinander Wasserstoff oder Niederalkyl bedeuten, m für 2 steht, n für 1 steht und $R_4$ und $R_6$ jeweils Wasserstoff bedeuten, in freier Form oder in Salzform.

Ganz besonders bevorzugt sind im Rahmen der Erfindung Verbindungen der Formel I, worin $R_1$, $R_2$, $R_3$, $R_5$ und $R_7$ jeweils Wasserstoff bedeuten, m für 2 steht, n für 1 steht und $R_4$ und $R_6$ entweder jeweils Wasserstoff bedeuten oder gemeinsam für eine zusätzliche Bindung stehen, in freier Form oder in Salzform.

In ganz besonderer Weise bevorzugt sind im Rahmen der Erfindung Verbindungen der Formel I, worin $R_1$, $R_2$, $R_3$, $R_5$ und $R_7$ jeweils Wasserstoff bedeuten, m für 2 steht, n für 1 steht und $R_4$ und $R_6$ jeweils Wasserstoff bedeuten, in freier Form oder in Salzform.

Namentlich bevorzugt sind im Rahmen der Erfindung die in den Beispielen genannten Verbindungen der Formel I in freier Form oder in Salzform.

Ebenfalls Gegenstand der vorliegenden Erfindung ist ein Verfahren zur Herstellung von Verbindungen der Formel I, z.B. dadurch gekennzeichnet, dass man

a) zur Herstellung von Verbindungen I, worin $R_4$ und $R_6$ gemeinsam für eine zusätzliche Bindung stehen, eine Verbindung der Formel

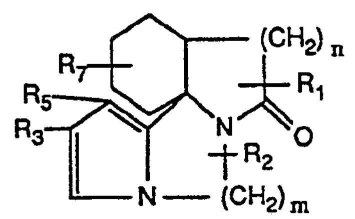

(II)

oder ein Salz davon oxidiert oder

b) zur Herstellung von Verbindungen I, worin $R_4$ und $R_6$ jeweils Wasserstoff bedeuten, in einer Verbindung der Formel

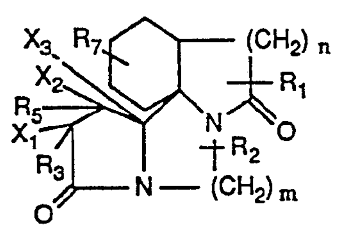

(III),

worin entweder $X_1$ und $X_2$ gemeinsam für eine zusätzliche Bindung stehen und $X_3$ Wasserstoff ist oder $X_1$ Wasserstoff ist und $X_2$ und $X_3$ gemeinsam für eine zusätzliche Bindung stehen, oder in einem Salz davon die Doppelbindung zwischen den die Reste $X_1$ und $X_2$ tragenden Kohlenstoffatomen oder zwischen den die Reste $X_2$ und $X_3$ tragenden Kohlenstoffatomen zu einer Einfachbindung reduziert und gewünschtenfalls jeweils ein verfahrensgemäss erhältliches Isomerengemisch in die Komponenten auftrennt und/oder eine verfahrensgemäss erhältliche freie Verbindung I in ein Salz überführt oder ein verfahrensgemäss erhältliches Salz in die freie Verbindung I oder in ein anderes Salz umwandelt.

Die vor- und nachstehend beschriebenen Umsetzungen werden in an sich bekannter Weise durchgeführt, z.B. in Ab- oder üblicherweise in Anwesenheit eines geeigneten Lösungsoder Verdünnungsmittels oder eines Gemisches derselben, wobei man je nach Bedarf unter Kühlen, bei Raumtemperatur oder unter Erwärmen, z.B. in einem Temperaturbereich von etwa -80° bis zur Siedetemperatur des Reaktionsmediums, vorzugsweise von etwa -20° bis etwa 150°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial, das für die Herstellung der Verbindungen I verwendet wird, ist bekannt oder kann nach an sich bekannten Methoden, z.B. gemäss den nachstehend beschriebenen Verfahrensweisen, hergestellt werden.

Für Salze von Ausgangsmaterialien gilt das vorstehend für Salze von Verbindungen I Gesagte in analoger Weise.

Die Oxidation gemäss der Verfahrensvariante a) kann nach verschiedenen Methoden durchgeführt werden.

Beispielsweise können Verbindungen II oder deren Salze durch Umsetzung mit einer Peroxoverbindung, z.B. mit Wasserstoffperoxid oder einem Salz davon, wie Natriumperoxid, Peroxodischwefelsäure oder einem Salz davon, wie Natriumperoxodisulfat, oder einer organischen Persäure, wie Perameisensäure, Peressigsäure oder einer gegebenenfalls substituierten, z.B. halogenierten, Perbenzoesäure, wie Perbenzoesäure oder m-Chlorperbenzoesäure, in Gegenwart eines Solvolysemittels, z.B. von Wasser, einem Alkohol, z.B. einem Niederalkanol, wie Aethanol, oder einer Säure, z.B. einer organischen Carbonsäure, wie einer gegebenenenfalls halogenierten Niederalkancarbonsäure, wie Ameisensäure, Essigsäure oder Trifluoressigsäure, oxidiert werden. Dabei sind, je nach Wahl der Oxidationsbedingungen, α,β-ungesättigte Lactame, β,γ-ungesättigte Lactame oder Gemische dieser beiden Lactamtypen (welche im allgemeinen in üblicher Weise, z.B. durch Chromatographie, in die Komponenten getrennt werden können) erhältlich. Geeignete Oxidationsbedingungen für die Bildung der gewünschten α,β-ungesättigten Lactame sind die üblicherweise für derartige Oxidationen gebräuchlichen Bedingungen, vorteilhaft die in den Beispielen angegebenen Bedingungen, wobei, falls Gemische der beiden erwähnten Lactam-Typen erhalten werden, aus diesen Gemischen das gewünschte α,β-ungesättigte Lactam abgetrennt wird.

Es können aber auch Verbindungen II oder deren Salze zunächst mit einem geeigneten Halogenierungsmittel, wie einem N-Halogensuccinimid, z.B. mit N-Bromsuccinimid oder N-Chlorsuccinimid, in die halogenierten Verbindungen der Formel

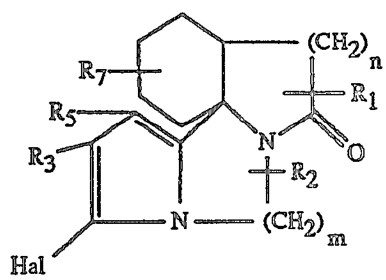

(IV),

worin Hal für Halogen, vorzugsweise Chlor oder insbesondere Brom, steht, oder deren Salze überführt werden und diese dann in einem zweiten Schritt zu Verbindungen I, worin $R_4$ und $R_6$ gemeinsam für eine zusätzliche Bindung stehen, z.B. mittels eines der vorstehend erwähnten Solvolysemittel, gegebenenfalls in Gegenwart geeigneter Katalysatoren, wie von Schwermetall-Katalysatoren, z.B. Platin- oder Palladium-Katalysatoren, wie von Tetrakis(triphenylphosphan)palladium(0), solvolysiert werden. Geeignete Halogenierungs- bzw. Solvolysebedingungen sind die üblicherweise für derartige Reaktionen gebräuchlichen Bedingungen, vorteilhaft die in den Beispielen angegebenen Bedingungen.

Verbindungen der Formel II und deren Salze sind bekannt oder können in Analogie zu bekannten Materialien hergestellt werden.

Die Ausgangsmaterialien III für die Verfahrensvariante b) können, je nach Position der zu reduzierenden Doppelbindung, entweder in Form der α,β-ungesättigten Lactame ($X_1$ + $X_2$ = Bindung ; $X_3$ = Wasserstoff) oder in Form der β,γ-ungesättigten Lactame ($X_1$ = Wasserstoff ; $X_2$ + $X_3$ = Bindung) oder in Form von Gemischen α,β-ungesättigter und β,γ-ungesättigter Lactame eingesetzt werden. Die α,β-ungesättigten Lactame sind identisch mit den gemäss der Verfahrensvariante a) erhältlichen Verbindungen I, worin $R_4$ und $R_6$ gemeinsam für eine zusätzliche Bindung stehen.

Die Reduktion der Kohlenstoff-Kohlenstoff-Doppelbindung in den ungesättigten Lactamen III zu einer Einfachbindung erfolgt in üblicher Weise durch Behandlung mit einem geeigneten Reduktionsmittel, beispielsweise durch Hydrierung in Gegenwart eines Hydrierungskatalysators, durch Reduktion mit einem Hydrid-übertragenden Reagens oder durch Reduktion mit einem metallischen Reduktionssystem aus Metall und Protonenabspaltendem Mittel.

Als Hydrierungskatalysatoren kommen z.B. Elemente der VIII. Nebengruppe des Periodensystems der Elemente oder deren Derivate in Betracht, wie Palladium, Platin, Platinoxid, Ruthenium, Rhodium, Tris(triphenylphosphan)rhodium(I)-halogenid, z.B. -chlorid, oder Raney-Nickel, die gegebenenfalls auf einem Trägermaterial, wie Aktivkohle, einem Alkalimetallcarbonat bzw. -sulfat oder einem Kieselgel, aufgezogen sind.

Als Hydrid-übertragende Reagentien kommen beispielsweise geeignete Leichtmetallhydride, insbesondere Alkalimetallaluminiumhydride bzw. -borhydride, wie Lithiumaluminiumhydrid, Lithiumtriäthylborhydrid, Natriumborhydrid, Natriumcyanoborhydrid, oder Zinnhydride, wie Triäthyl- oder Tributylzinnhydrid, oder Diboran in Frage. Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Uebergangsmetall, z.B. Zink, Zinn, Eisen oder Titan, während als Protonen-abspaltende Mittel z.B. Protonensäuren der vorstehend bei der Definition der Säureadditionssalze genannten Art, wie Salz- oder Essigsäure, Niederalkanole, wie Aethanol, und/oder Amine bzw. Ammoniak in Frage kommen. Solche Systeme sind beispielsweise Natrium/Ammoniak, Zink/Salz- oder Essigsäure oder Zink/Aethanol.

Die Reduktion von Verbindungen III und deren Salzen erfolgt z.B. in Gegenwart eines geeigneten inerten Lösungs- oder Verdünnungsmittels, wie eines gegebenenfalls halogenierten Kohlenwasserstoffs, z.B. von Hexan, Cyclohexan, Benzol, Toluol, Dichlormethan oder Chlorbenzol, eines Aethers, z.B. von Diäthyläther, Dioxan oder Tetrahydrofuran, oder eines Ketons, z.B. von Aceton oder 2-Butanon, und bei Raumtemperatur oder unter schwachem Erwärmen, z.B. in einem Temperaturbereich von etwa 20° bis etwa 100°C.

Besonders bevorzugte Ausfürungsformen des Reduktionsverfahrens sind in den Beispielen dargestellt.

Die Herstellung der Ausgangsmaterialien III und ihrer Salze erfolgt in üblicher Weise, z.B. durch Oxidation von Verbindungen II oder deren Salzen, wobei in analoger Weise wie unter der Verfahrensvariante a) erläutert gearbeitet wird und die Oxidationsbedingungen jeweils so gewählt werden, dass der (die) gewünschte(n) Typ(en) der möglichen Lactame erhalten wird (werden). Geeignete Oxidationsbedingungen sind die üblicherweise für derartige Oxidationen gebräuchlichen Bedingungen, vorteilhaft die in den Beispielen angegebenen Bedingungen.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Infolge der engen Beziehung zwischen der Verbindung I in freier Form und in Form ihrer Salze sind im vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren oder als Gemisch derselben vorliegen. Dabei sind, in Abhängigkeit von der Molekülsymmetrie, z.B. je nach Anzahl, absoluter und relativer Konfiguration der Chiralitätszentren, wie asymmetrischen C-Atome, als reine Isomere z.B. reine Enantiomere und/oder reine Diastereomere, wie reine cis/trans-Isomere oder meso-Verbindungen, erhältlich. Entsprechend können als Isomerengemische z.B. Enantiomerengemische, wie Racemate, Diastereomerengemische oder Racematgemische vorliegen. Verfahrensgemäss oder anderweitig erhältliche Isomerengemische von Verbindungen I in freier Form oder in Salzform können in üblicher Weise in die Komponenten aufgetrennt werden.

Erhaltene Diastereomerengemische und Racematgemische können aufgrund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Erhaltene Enantiomerengemische, wie Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Enantiomeren verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I führen. Neue Ausgangsstoffe und Zwischenprodukte, jeweils in freier Form oder in Salzform, für die Herstellung der Verbindungen I, ihre Verwendung und Verfahren zu ihrer Herstellung bilden ebenfalls einen Gegenstand der Erfindung, wobei die Variablen $R_1$, $R_2$, $R_3$, $R_4$, $R_5$, $R_6$, $R_7$, m und n die für die Verbindungen I angegebenen Bedeutungen haben.

Die Erfindung betrifft ebenfalls die Verwendung der Verbindungen I und ihrer pharmazeutisch verwendbaren Salze zur Behandlung von cerebralen Insuffizienzerscheinungen, insbesondere Gedächtnisstörungen, vorzugsweise in Form von pharmazeutisch verwendbaren Zubereitungen, insbesondere in einem Verfahren zur therapeutischen Behandlung des tierischen oder menschlichen Körpers, sowie ein solches Behandlungsverfahren.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich um solche zur enteralen, wie oralen, ferner rektalen oder parenteralen Verabreichung an Warmblüter, wobei der pharmakologische Wirkstoff allein oder zusammen mit üblichen pharmazeutischen Hilfsstoffen enthalten ist. Die pharmazeutischen Präparate enthalten z.B. von etwa 0,1% bis 100%, vorzugsweise von etwa 1% bis etwa 50%, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten, Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffs kann von verschiedenen Faktoren, wie Applikationsweise, Warmblüter-Spezies, Alter und/oder individuellem Zustand, abhängen. Im Normalfall ist für einen etwa 75 kg schweren Warmblüter bei oraler Applikation eine ungefähre Tagesdosis von etwa 20 mg bis etwa 1500 mg, insbesondere von etwa 50 bis etwa 250 mg, vorteilhaft in mehreren gleichen Teildosen, zu veranschlagen.

Die nachfolgenden Beispiele illustrieren die oben beschriebene Erfindung ; sie sollen jedoch diese in ihrem Umfang in keiner Weise einschränken. Temperaturen sind in Celsiusgraden angegeben.

Beispiel 1 :

a) 700 mg (2,8 mmol) (9aR*,9bR*,13aR*)-2,7-Dioxo-1,4,5,7,9a,10,11,12,13,13a-dekahydro-2H-pyrrolo[2',1' : 3,4]pyrazino[2,1-i]indol, gelöst in 30 ml Tetrahydrofuran, werden in Gegenwart von 70 mg $PtO_2$ mit Wasserstoff bei Raumtemperatur unter Normaldruck bis zum Ende der Wasserstoffaufnahme hydriert. Der Katalysator wird abfiltriert und das Filtrat im Vakuum eingedampft. Das resultierende Oel kristallisiert man aus Aethylacetat. Man erhält so das (9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol der Formel

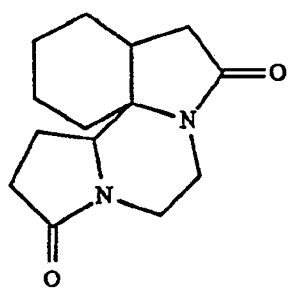

(I'),

also die Verbindung der Formel I, in der $R_1$, $R_2$, $R_3$, $R_4$, $R_5$, $R_6$ und $R_7$ jeweils ein Wasserstoffatom bedeuten, m für 2 steht, n für 1 steht und die chiralen C-Atome 9a, 9b und 13a die vorstehende relative Konfiguration (jeweils R*) aufweisen (Schmelzbereich : 135 bis 136°). Die Mutterlauge wird im Vakuum eingedampft und an Kieselgel (0,040-0,063 mm) mit Trichlormethan bzw. Trichlormethan/Methanol (99 : 1) als Laufmittel chromatographiert. Man erhält so das hinsichtlich der relativen Konfiguration des C-Atoms 9a stereoisomere (9aR*,9bS*,13aS*)-2,7-Dioxo-1,4,5,7,8,9,9a,10,11,12,13,13a-dodekahydro-2H-pyrrolo[2',1' : 3,4]pyrazino[2,1-i]indol in Form eines Oels, das aus Aethylacetat kristallisiert wird und dann bei 177 bis 179° schmilzt.

b) Das als Ausgangsmaterial verwendete α,β-ungesättigte Lactam kann man z.B. wie folgt herstellen :
Eine Lösung von 2,3 g (10 mmol) (9bR*,13aR*)-2-Oxo-1,4,5,10,11,12,13,13a-oktahydro-2H-pyrrolo[2',1' : 3,4]pyrazino[2,1-i]indol in 50 ml Dichlormethan wird unter Rühren bei Raumtemperatur portionsweise mit 4,05 g (20 mmol) m-Chlorperbenzoesäure versetzt (die exotherme Reaktion wird mit einem Eisbad bei Raumtemperatur gehalten). Anschliessend tropft man zu dem Reaktionsgemisch 2,28 g (20 mmol) Trifluoressigsäure zu und rührt 16 Stunden bei Raumtemperatur nach. Das Reaktionsgemisch wird mit 100 ml Dichlormethan verdünnt und dann nacheinander mit Natriumhydrogensulfitlösung (5%) und Natriumhydrogencarbonatlösung (5%) gewaschen. Die organische Phase wird abgetrennt, über Natriumsulfat getrocknet und im Vakuum eingedampft. Das so erhältliche Rohprodukt wird an 210 g Kieselgel (0,040-0,063 mm) mit Trichlormethan als Laufmittel chromatographiert. Man erhält so das (9aR*,9bR*,13aR*)-2,7-Dioxo-1,4,5,7,9a,10,11,12,13,13a-dekahydro-2H-pyrrolo[2',1' : 3,4]pyrazino[2,1-i]indol in Form eines Oels, das aus Dichlormethan/Diäthyläther kristallisiert wird und bei 159 bis 162° schmilzt.

c) Das als Ausgangsmaterial verwendete α,β-ungesättigte Lactam kann z.B. auch wie folgt hergestellt werden :
2,3 g (10 mmol) (9bR*,13aR*)-2-Oxo-1,4,5,10,11,12,13,13a-oktahydro-2H-pyrrolo[2',1' : 3,4]pyrazino[2,1-i]indol werden in 60 ml absolutem Tetrahydrofuran gelöst. Die Lösung wird bei 0° portionsweise mit 1,78 g (10 mmol) N-Bromsuccinimid versetzt. Das Reaktionsgemisch wird dann 30 Minuten bei 0° gerührt.

Anschliessend lässt man es 12 Stunden bei Raumtemperatur stehen. Die rötliche Mischung wird im Vakuum weitgehend eingeengt, der Rückstand mit 100 ml Diäthyläther und 100 ml Wasser versetzt, das Gemisch ausgeschüttelt und die organische Phase abgetrennt, nacheinander mit 30 ml 0,5M-Natriumhydrogensulfitlösung und 1N-Natriumhydrogencarbonatlösung gewaschen, über Magnesiumsulfat getrocknet und vom Lösungsmittel befreit. Der Rückstand wird zweimal aus Diäthyläther/Pentan kristallisiert. Man erhält so ein Gemisch, bestehend aus 87% (9bR*,13aR*)-7-Brom-2-oxo-1,4,5,10,11,12,13,13a-oktahydro-2H-pyrrolo[2',1' : 3,4]pyrazino[2,1-i]indol und 13% (9bR*,13aR*)-7,8-Dibrom-2-oxo-1,4,5,10,11,12,13,13a-oktahydro-2H-pyrrolo[2',1' : 3,4]pyrazino[2,1-i]indol (Schmelzbereich des Gemisches : 118 bis 120°), das ohne weitere Reinigung weiterverwendet wird.

0,618 g (2 mmol) dieses Bromidgemisches und 0,820 g (10 mmol) wasserfreies Natriumacetat werden bei Raumtemperatur in 40 ml Eisessig gelöst. Die Lösung wird unter Argon mit 0,28 g (0,24 mmol) Tetrakis(triphenylphosphan)palladium(0) versetzt. Das Reaktionsgemisch wird 6 Stunden bei 100° gerührt, dann abgekühlt und im Vakuum vom Lösungsmittel befreit. Der Rückstand wird in Dichlormethan aufgenommen und die ungelösten Anteile werden über Celite abfiltriert. Das Filtrat wird mit 1N-Natriumhydrogencarbonatlösung gewaschen und über Magnesiumsulfat getrocknet Nach Entfernen des Lösungsmittels hinterbleibt ein rotes viskoses Oel, aus dem durch präparative Dünnschichtchromatographie [Silicagel ; Laufmittel : Toluo/Aethanol/konzentriertes Ammoniak (90 : 20 : 1)] das reine (9aR*,9bR*,13aR*)-2,7-Dioxo-1,4,5,7,9a,10,11,12,13,13a-dekahydro-2H-pyrrolo[2',1' : 3,4]pyrazino[2,1-i]indol abgetrennt werden kann, das nach Umkristallisation aus Aethylacetat/Diäthyläther/Pentan bei 160 bis 162° schmilzt.

<u>Beispiel 2 :</u>

985 mg (4 mmol) (9bR*,13aR*)-2,7-Dioxo-1,4,5,7,8,10,11,12,13,13a-dekahydro-2H-pyrrolo[2',1' : 3,4]pyrazino[2,1-i]indol, gelöst in 40 ml Tetrahydrofuran, werden in Gegenwart von 98 mg $PtO_2$ mit Wasserstoff bei Raumtemperatur unter Normaldruck bis zum Ende der Wasserstoffaufnahme hydriert. Der Katalysator wird abfiltriert und das Filtrat im Vakuum eingedampft. Das resultierende ölige Rohprodukt kristallisiert man aus Aethylacetat. Man erhält so das (9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol, das bei 135 bis 136° schmilzt. Die Mutterlauge wird im Vakuum eingedampft und an Kieselgel (0,040-0,063 mm) mit Trichlormethan bzw. Trichlormethan/Methanol (99 : 1) als Laufmittel chromatographiert. Man erhält so das hinsichtlich der relativen Konfiguration des C-Atoms 9a stereoisomere (9aR*,9bS*,13aS*)-2,7-Dioxo-1,4,5,7,8,9,9a,10,11,12,13,13a-dodekahydro-2H-pyrrolo[2',1' : 3,4]pyrazino[2,1-i]indol in Form eines Oels, das aus Aethylacetat kristallisiert wird und dann bei 177 bis 179° schmilzt.

Das als Ausgangsmaterial verwendete β,γ-ungesättigte Lactam kann z.B. folgendermassen hergestellt werden :

Zu einer Lösung von 2,3 g (10 mmol) (9bR*,13aR*)-2-Oxo-1,4,5,10,11,12,13,13a-oktahydro-2H-pyrrolo[2',1' : 3,4]pyrazino[2,1-i]indol in 30 ml Dichlormethan wird innert 45 Minuten unter Rühren bei -15° eine Lösung von 3,04 g (15 mmol) m-Chlorperbenzoesäure in 30 ml Dichlormethan zugetropft. Anschliessend wird das Reaktionsgemisch 4 Stunden bei -15° nachgerührt, dann mit 100 ml Dichlormethan verdünnt und nacheinander eiskalt mit Natriumhydrogensulfitlösung (5%) und Natriumhydrogencarbonatlösung (5%) gewaschen. Die organische Phase wird abgetrennt, über Natriumsulfat getrocknet und im Vakuum eingedampft. Man erhält so ein Rohprodukt, das an 120 g Kieselgel (0,040-0,063 mm) mit Trichlormethan als Laufmittel chromatographiert wird. Aus den entsprechenden Fraktionen erhält man das gesuchte (9bR*,13aR*)-2,7-Dioxo-1,4,5,7,8,10,11,12,13,13a-dekahydro-2H-pyrrolo[2',1' : 3,4]pyrazino[2,1-i]indol in Form eines Schaumes, das aus Dichlormethan/Diäthyläther kristallisiert wird und dann bei 105 bis 125° schmilzt. Man erhält auch das isomere (9aR*,9bR*,13aR*)-2,7-Dioxo-1,4,5,7,9a,10,11,12,13,13a-dekahydro-2H-pyrrolo[2',1' : 3,4]pyrazino[2,1-i]indol in Form eines Oels, das aus Dichlormethan/Diäthyläther kristallisiert wird und dann bei 159 bis 162° schmilzt.

<u>Beispiel 3 :</u>

Zu einer Lösung von 2,3 g (10 mmol) (+)-(9bR*,13aR*)-2-Oxo-1,4,5,10,11,12,13,13a-oktahydro-2H-pyrrolo[2',1' : 3,4]pyrazino[2,1-i]indol in 30 ml Dichlormethan wird innert 45 Minuten unter Rühren bei -15° eine Lösung von 3,04 g (15 mmol) m-Chlorperbenzoesäure in 30 ml Dichlormethan zugetropft. Anschliessend wird das Reaktionsgemisch 4 Stunden bei -15° nachgerührt, dann mit 100 ml Dichlormethan verdünnt und nacheinander eiskalt mit Natriumhydrogensulfitlösung (5%) und Natriumhydrogencarbonatlösung (5%) gewaschen. Die organische Phase wird abgetrennt, über Natriumsulfat getrocknet und im Vakuum eingedampft. Man erhält das (+)-(9bR*,13aR*)-2,7-Dioxo-1,4,5,7,8,10,11,12,13,13a-dekahydro-2H-pyrrolo[2',1' : 3,4]pyrazino[2,1-i]indol in Form eines Schaumes, der ohne weitere Reinigung weiterumgesetzt werden kann. Das (-)-(9bR*,13aR*)-2,7-Dioxo-1,4,5,7,8,10,11,12,13,13a-dekahydro-2H-pyrrolo[2',1' : 3,4]pyrazino[2,1-i]indol wird in analoger Weise hergestellt, ausgehend von 2,3 g (10 mmol) (-)-(9bR*,13aR*)-2-Oxo-1,4,5,10,11,12,13,13a-oktahydro-2H-pyrrolo[2',1' : 3,4]pyrazino[2,1-i]indol, und in Form eines Schaumes erhalten, der ohne weitere Reinigung weiterumgesetzt werden kann.

Die Ausgangsmaterialien kann man z.B. wie folgt herstellen :

11,0 g (±)-(9bR*,13aR*)-2-Oxo-1,4,5,10,11,12,13,13a-oktahydro-2H-pyrrolo[2',1' : 3,4]pyrazino[2,1-i]indol werden in 22 Portionen von je 0,5 g an einer Büchi-Glassäule (600 × 50 mm) mit Tribenzoylcellulose als Füllmaterial bei ungefähr 7 bar mit Hexan/Isopropanol (9 : 1) als Laufmittel chromatographiert [Detektion : UV (230 nm)]. Man erhält nach dem Eindampfen der Spitzenfraktionen das rohe (+)-Enantiomere in Form eines Oels, das aus Diäthyläther/Petroläther kristallisiert. Nach dem Umkristallisieren aus Diäthyläther/Petroläther erhält man das reine (+)-(9bR*, 13aR*)-2-Oxo-1,4,5,10,11,12,13,13a-oktahydro-2H-pyrrolo[2',1' : 3,4]pyrazino[2,1-i-]indol, das bei 122 bis 123° schmilzt {$[\alpha]_D^{20}$ = +153,7° ±1,0° ($CHCl_3$ ; c = 1)}. Nach Abtrennung der Mischfraktionen eluiert man am Schluss das (-)-Enantiomere. Man erhält nach dem Eindampfen der Schlussfraktionen das (-)-Enantiomere in Form eines Oels, das aus Diäthyläther/Petroläther kristallisiert. Nach Umkristallisation aus Diäthyläther/Petroläther erhält man das reine (-)-(9bR*,13aR*)-2-Oxo-1,4,5,10,11,12,13,13a-oktahydro-2H-pyrrolo[2',1' : 3,4]pyrazino[2,1-i]indol, das bei 122 bis 123° schmilzt {$[\alpha]_D^{20}$ = -154,8° ±1,0° ($CHCl_3$ ; c = 1)}.

<u>Beispiel 4 :</u>

2,21 g (9 mmol) (+)-(9bR*,13aR*)-2,7-Dioxo-1,4,5,7,8,10,11,12,13,13a-dekahydro-2H-pyrrolo[2',1' : 3,4]pyrazino[2,1-i]indol, gelöst in 100 ml Tetrahydrofuran, werden in Gegenwart von 220 mg $PtO_2$ mit Wasserstoff bei Raumtemperatur unter Normaldruck bis zum Ende der Wasserstoffaufnahme hydriert. Der Katalysator wird abfiltriert und das Filtrat im Vakuum eingedampft. Der ölige Rückstand wird in Dichlormethan gelöst und die Lösung dann nacheinander mit 0,1 N-Salzsäure und Natriumhydrogencarbonatlösung (5%) gewaschen. Die organische Phase wird über Natriumsulfat getrocknet und im Vakuum eingedampft. Man erhält so ein Rohprodukt, das an 60 g Kieselgel (0,040-0,063 mm) mit Dichlormethan bzw. Dichlormethan/Methanol (99 : 1) als Laufmittel chromatographiert wird. Aus den entsprechenden Fraktionen erhält man das gewünschte Produkt in roher Form (Oel). Das Oel wird in 50 ml Wasser gelöst und die Lösung zweimal mit je 50 ml Diäthyläther extrahiert. Die wässrige Phase wird bei Raumtemperatur mit Entfärbungskohle behandelt und dann im Vakuum eingedampft. Der Rückstand wird 36 Stunden im Hochvakuum getrocknet. Man erhält so das reine (+)-(9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol in Form eines farblosen Oels, das 0,64 Aequivalente Wasser enthält {$[\alpha]_D^{20}$ = +53,7° ±1,0° ($CHCl_3$ ; c = 1)}.

Das (-)-(9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol wird in analoger Weise hergestellt, ausgehend von 1,97 g (8 mmol) (-)-(9bR*,13aR*)-2,7-Dioxo-1,4,5,7,8,10,11,12,13,13a-dekahydro-2H-pyrrolo[2',1' : 3,4]pyrazino[2,1-i]indol, und in Form eines farblosen Oels erhalten, das 0,61 Aequivalente Wasser enthält {$[\alpha]_D^{20}$ = -52,1° ±1,0° ($CHCl_3$ ; c = 1)}.

<u>Beispiel 5 :</u>

Tabletten, enthaltend 25 mg des Wirkstoffs, z.B. (9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]-indol, können folgendermassen hergestellt werden :

<u>Bestandteile (für 1000 Tabletten) :</u>

| | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

<u>Herstellung :</u>

Sämtliche festen Ingredienzien werden zunächst durch ein Sieb mit 0,6 mm Maschenweite getrieben. Dann werden der Wirkstoff, die Lactose, das Talkum, das Magnesiumstearat und die Hälfte der Stärke vermischt.

Die andere Hälfte der Stärke wird in 40 ml Wasser suspendiert und diese Suspension zu einer siedenden Lösung des Polyäthylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 6 :

Tabletten, enthaltend 50 mg des Wirkstoffs, z.B. (9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol, werden wie folgt hergestellt :

Zusammensetzung (für 10000 Tabletten) :

| | |
|---|---|
| Wirkstoff | 500,00 g |
| Lactose | 140,80 g |
| Kartoffelstärke | 274,70 g |
| Stearinsäure | 10,00 g |
| Talk | 50,00 g |
| Magnesiumstearat | 2,50 g |
| Kolloidales Siliciumdioxid | 32,00 g |
| Aethanol | q.s. |

Ein Gemisch des Wirkstoffs, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen sein können.

In analoger Weise können 100 mg Wirkstoff eingearbeitet werden.

Beispiel 7 :

Kapseln, enthaltend 0,025 g des Wirkstoffs, z.B. (9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol, können wie folgt hergestellt werden :

Zusammensetzung (für 1000 Kapseln) :

| | |
|---|---|
| Wirkstoff | 25,00 g |
| Lactose | 249,00 g |
| Gelatine | 2,00 g |
| Maisstärke | 10,00 g |
| Talk | 15,00 g |
| Wasser | q.s. |

Man mischt den Wirkstoff mit der Lactose, befeuchtet die Mischung gleichmässig mit einer wässrigen Lösung der Gelatine und granuliert sie durch ein Sieb mit einer Maschenweite von 1,2 bis 1,5 mm. Das Granulat mischt man mit der getrockneten Maisstärke und dem Talk und füllt Portionen von 300 mg in Hartgelatinekapseln (Grösse 1) ab.

Beispiel 8 :

In analoger Weise wie in den Beispielen 5 bis 7 beschrieben können auch pharmazeutische Präparate, als Wirkstoff enthaltend eine andere Verbindung I oder ein pharmazeutisch verwendbares Salz einer Verbindung I, beispielsweise gemäss den Beispielen 1 bis 4, hergestellt werden.

## Ansprüche

1. Eine Verbindung der Formel

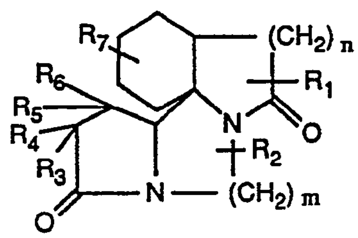

(I),

worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ entweder jeweils Wasserstoff bedeuten oder gemeinsam für eine zusätzliche Bindung stehen, in freier Form oder in Salzform.

2. Eine Verbindung gemäss Anspruch 1 der Formel I, worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ jeweils Wasserstoff bedeuten, in freier Form oder in Salzform.

3. Eine Verbindung gemäss Anspruch 1 der Formel I, worin $R_1$, $R_2$, $R_3$, $R_5$ und $R_7$ jeweils unabhängig voneinander Wasserstoff oder Niederalkyl bedeuten, m für 2 steht, n für 1 steht und $R_4$ und $R_6$ entweder jeweils Wasserstoff bedeuten oder gemeinsam für eine zusätzliche Bindung stehen, in freier Form oder in Salzform.

4. Eine Verbindung gemäss Anspruch 1 der Formel I, worin $R_1$, $R_2$, $R_3$, $R_5$ und $R_7$ jeweils unabhängig voneinander Wasserstoff oder Niederalkyl bedeuten, m für 2 steht, n für 1 steht und $R_4$ und $R_6$ jeweils Wasserstoff bedeuten, in freier Form oder in Salzform.

5. Eine Verbindung gemäss Anspruch 1 der Formel I, worin $R_1$, $R_2$, $R_3$, $R_5$ und $R_7$ jeweils Wasserstoff bedeuten, m für 2 steht, n für 1 steht und $R_4$ und $R_6$ entweder jeweils Wasserstoff bedeuten oder gemeinsam für eine zusätzliche Bindung stehen, in freier Form oder in Salzform.

6. Eine Verbindung gemäss Anspruch 1 der Formel I, worin $R_1$, $R_2$, $R_3$, $R_5$ und $R_7$ jeweils Wasserstoff bedeuten, m für 2 steht, n für 1 steht und $R_4$ und $R_6$ jeweils Wasserstoff bedeuten, in freier Form oder in Salzform.

7. (9aR*,9bR*,13aR*)-2,7-Dioxo-1,4,5,7,8,9,9a,10,11,12,13, 13a-dodekahydro-2H-pyrrolo[2',1': 3,4]pyrazino[2,1-i]indol in freier Form oder in Salzform.

8. (9aR*,9bR*,13aR*)-2,7-Dioxo-1,4,5,7,9a,10,11,12,13,13a-dekahydro-2H-pyrrolo[2',1': 3,4]pyrazino[2,1-i]indol in freier Form oder in Salzform.

9. (+)-(9aR*,9bR*,13aR*)-2,7-Dioxo-1,4,5,7,8,9,9a,10,11,12,13,13a-dodekahydro-2H-pyrrolo[2',1': 3,4]pyrazino[2,1-i]indol in freier Form oder in Salzform.

10. (-)-(9aR*,9bR*,13aR*)-2,7-Dioxo-1,4,5,7,8,9,9a,10,11,12,13,13a-dodekahydro-2H-pyrrolo[2',1': 3,4]pyrazino[2,1-i]indol in freier Form oder in Salzform.

11. (9aR*,9bS*,13aS*)-2,7-Dioxo-1,4,5,7,8,9,9a,10,11,12,13,13a-dodekahydro-2H-pyrrolo[2',1': 3,4]pyrazino[2,1-i]indol in freier Form oder in Salzform.

12. Eine Verbindung gemäss einem der Ansprüche 1 bis 11 in freier Form oder in pharmazeutisch verwendbarer Salzform zur Anwendung in einem Verfahren zur therapeutischen Behandlung des menschlichen oder tierischen Körpers.

13. Eine Verbindung gemäss einem der Ansprüche 2, 4, 6 bis 8 und 11 in freier Form oder in pharmazeutisch verwendbarer Salzform zur Anwendung in einem Verfahren zur therapeutischen Behandlung des menschlichen oder tierischen Körpers.

14. Eine Verbindung gemäss einem der Ansprüche 1 bis 13 in freier Form oder in pharmazeutisch verwendbarer Salzform zur Anwendung als Nootropikum.

15. Eine Verbindung gemäss einem der Ansprüche 2, 4, 6 bis 8, 11 und 13 in freier Form oder in pharmazeutisch verwendbarer Salzform zur Anwendung als Nootropikum.

16. Ein pharmazeutisches Präparat, als Wirkstoff enthaltend eine Verbindung gemäss einem der Ansprüche 1 bis 15 in freier Form oder in pharmazeutisch verwendbarer Salzform, gegebenenfalls neben üblichen pharmazeutischen Hilfsstoffen.

17. Ein pharmazeutisches Präparat, als Wirkstoff enthaltend eine Verbindung gemäss einem der Ansprüche 2, 4, 6 bis 8, 11, 13 und 15 in freier Form oder in pharmazeutisch verwendbarer Salzform, gegebenenfalls neben üblichen pharmazeutischen Hilfsstoffen.

18. Ein nootrop wirksames pharmazeutisches Präparat gemäss Anspruch 16 oder 17, dadurch gekennzeichnet, dass man einen nootrop wirksamen Wirkstoff wählt.

19. Ein nootrop wirksames pharmazeutisches Präparat gemäss Anspruch 17, dadurch gekennzeichnet, dass man einen nootrop wirksamen Wirkstoff wählt.

20. Verfahren zur Herstellung einer Verbindung der Formel

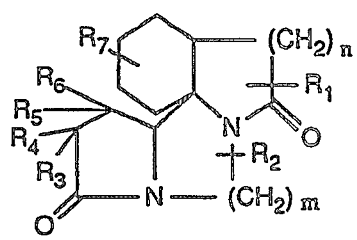

(I),

worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ entweder jeweils Wasserstoff bedeuten oder gemeinsam für eine zusätzliche Bindung stehen, in freier Form oder in Salzform, dadurch gekennzeichnet, dass man

a) zur Herstellung einer Verbindung I, worin $R_4$ und $R_6$ gemeinsam für eine zusätzliche Bindung stehen, eine Verbindung der Formel

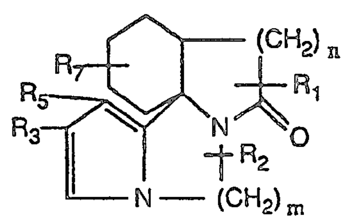

(II)

oder ein Salz davon oxidiert oder

b) zur Herstellung einer Verbindung I, worin $R_4$ und $R_6$ jeweils Wasserstoff bedeuten, in einer Verbindung der Formel

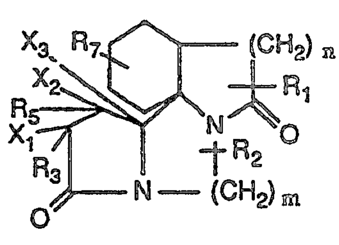

(III),

worin entweder $X_1$ und $X_2$ gemeinsam für eine zusätzliche Bindung stehen und $X_3$ Wasserstoff ist oder $X_1$ Wasserstoff ist und $X_2$ und $X_3$ gemeinsam für eine zusätzliche Bindung stehen, oder in einem Salz davon die Doppelbindung zwischen den die Reste $X_1$ und $X_2$ tragenden Kohlenstoffatomen oder zwi-

schen den die Reste $X_2$ und $X_3$ tragenden Kohlenstoffatomen zu einer Einfachbindung reduziert und gewünschtenfalls jeweils ein verfahrensgemäss erhältliches Isomerengemisch in die Komponenten auftrennt und/oder eine verfahrensgemäss erhältliche freie Verbindung I in ein Salz überführt oder ein verfahrensgemäss erhältliches Salz in die freie Verbindung I oder in ein anderes Salz umwandelt.

21. Verfahren zur Herstellung eines pharmazeutischen Präparates gemäss einem der Ansprüche 16 bis 19, dadurch gekennzeichnet, dass man den Wirkstoff, gegebenenfalls unter Beimischung von üblichen pharmazeutischen Hilfsstoffen, zu einem pharmazeutischen Präparat verarbeitet.

22. Verfahren gemäss Anspruch 21 zur Herstellung eines nootrop wirksamen pharmazeutischen Präparates gemäss Anspruch 18 oder 19, dadurch gekennzeichnet, dass man einen nootrop wirksamen Wirkstoff wählt.

23. Verwendung einer Verbindung gemäss einem der Ansprüche 1 bis 15 in freier Form oder in pharmazeutisch verwendbarer Salzform zur Herstellung eines pharmazeutischen Präparats.

24. Verwendung einer Verbindung gemäss einem der Ansprüche 1 bis 15 in freier Form oder in pharmazeutisch verwendbarer Salzform zur Herstellung eines pharmazeutischen Präparats auf nicht-chemischem Wege.

25. Verwendung einer Verbindung gemäss Anspruch 23 oder 24 zur Herstellung eines Nootropikums.

**Ansprüche für folgenden Vertragsstaaten : ES und GR**

1. Verfahren zur Herstellung einer Verbindung der Formel

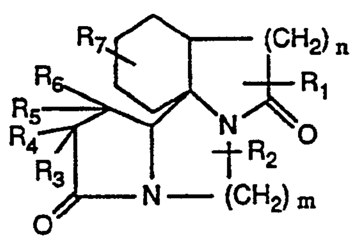

(I),

worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ entweder jeweils Wasserstoff bedeuten oder gemeinsam für eine zusätzliche Bindung stehen, in freier Form oder in Salzform, dadurch gekennzeichnet, dass man

a) zur Herstellung einer Verbindung I, worin $R_4$ und $R_6$ gemeinsam für eine zusätzliche Bindung stehen, eine Verbindung der Formel

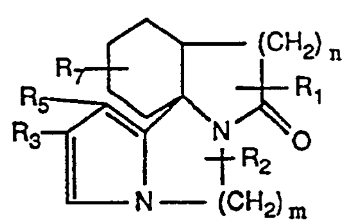

(II)

oder ein Salz davon oxidiert oder

b) zur Herstellung einer Verbindung I, worin $R_4$ und $R_6$ jeweils Wasserstoff bedeuten, in einer Verbindung der Formel

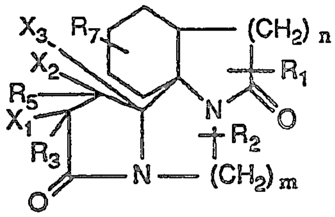

(III),

worin entweder $X_1$ und $X_2$ gemeinsam für eine zusätzliche Bindung stehen und $X_3$ Wasserstoff ist oder $X_1$ Wasserstoff ist und $X_2$ und $X_3$ gemeinsam für eine zusätzliche Bindung stehen, oder in einem Salz davon die Doppelbindung zwischen den die Reste $X_1$ und $X_2$ tragenden Kohlenstoffatomen oder zwischen den die Reste $X_2$ und $X_3$ tragenden Kohlenstoffatomen zu einer Einfachbindung reduziert und gewünschtenfalls jeweils ein verfahrensgemäss erhältliches Isomerengemisch in die Komponenten auftrennt und/oder eine verfahrensgemäss erhältliche freie Verbindung I in ein Salz überführt oder ein verfahrensgemäss erhältliches Salz in die freie Verbindung I oder in ein anderes Salz umwandelt.

2. Verfahren gemäss Anspruch 1 zur Herstellung einer Verbindung der Formel I, worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ jeweils Wasserstoff bedeuten, in freier Form oder in Salzform.

3. Verfahren gemäss Anspruch 1 zur Herstellung einer Verbindung der Formel I, worin $R_1$, $R_2$, $R_3$, $R_5$ und $R_7$ jeweils unabhängig voneinander Wasserstoff oder Niederalkyl bedeuten, m für 2 steht, n für 1 steht und $R_4$ und $R_6$ entweder jeweils Wasserstoff bedeuten oder gemeinsam für eine zusätzliche Bindung stehen, in freier Form oder in Salzform.

4. Verfahren gemäss Anspruch 1 zur Herstellung einer Verbindung der Formel I, worin $R_1$, $R_2$, $R_3$, $R_5$ und $R_7$ jeweils unabhängig voneinander Wasserstoff oder Niederalkyl bedeuten, m für 2 steht, n für 1 steht und $R_4$ und $R_6$ jeweils Wasserstoff bedeuten, in freier Form oder in Salzform.

5. Verfahren gemäss Anspruch 1 zur Herstellung einer Verbindung der Formel I, worin $R_1$, $R_2$, $R_3$, $R_5$ und $R_7$ jeweils Wasserstoff bedeuten, m für 2 steht, n für 1 steht und $R_4$ und $R_6$ entweder jeweils Wasserstoff bedeuten oder gemeinsam für eine zusätzliche Bindung stehen, in freier Form oder in Salzform.

6. Verfahren gemäss Anspruch 1 zur Herstellung einer Verbindung der Formel I, worin $R_1$, $R_2$, $R_3$, $R_5$ und $R_7$ jeweils Wasserstoff bedeuten, m für 2 steht, n für 1 steht und $R_4$ und $R_6$ jeweils Wasserstoff bedeuten, in freier Form oder in Salzform.

7. Verfahren gemäss Anspruch 1 zur Herstellung von (9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol in freier Form oder in Salzform.

8. Verfahren gemäss Anspruch 1 zur Herstellung von (9aR*,9bR*, 13aR*)-2,7-Dioxo-1,4,5,7,9a,10,11,12,13,13a-dekahydro-2H-pyrrolo[2',1' : 3,4]pyrazino[2,1-i]indol in freier Form oder in Salzform.

9. Verfahren gemäss Anspruch 1 zur Herstellung von (+)-(9aR*,9bR*, 13aR*)-2,7-Dioxo-1,4,5,7,8,9,9a,10,11,12,13,13a-dodekahydro-2H-pyrrolo[2',1' : 3,4]pyrazino[2,1-i] indol in freier Form oder in Salzform.

10. Verfahren gemäss Anspruch 1 zur Herstellung von (-)-(9aR*,9bR*,13aR*)-2,7-Dioxo-1,4,5,7,8,9,9a,10,11,12,13,13a-dodekahydro-2H-pyrrolo[2',1' : 3,4]pyrazino[2,1-i]indol in freier Form oder in Salzform.

11. Verfahren gemäss Anspruch 1 zur Herstellung von (9aR*,9bS*,13aS*)-2,7-Dioxo-1,4,5,7,8,9,9a,10,11,12,13,13a-dodekahydro-2H-pyrrolo[2', 1' : 3,4]pyrazino[2,1-i]indol in freier Form oder in Salzform.

12. Verfahren zur Herstellung eines pharmazeutischen Präparats, dadurch gekennzeichnet, dass man a) zur Herstellung einer Verbindung I, worin $R_4$ und $R_6$ gemeinsam für eine zusätzliche Bindung stehen, eine Verbindung der Formel

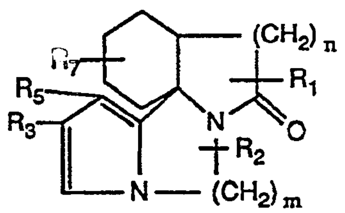

(II)

oder ein Salz davon oxidiert oder

b) zur Herstellung einer Verbindung I, worin $R_4$ und $R_6$ jeweils Wasserstoff bedeuten, in einer Verbindung der Formel

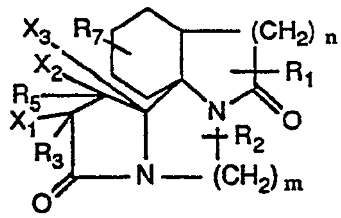

(III),

worin entweder $X_1$ und $X_2$ gemeinsam für eine zusätzliche Bindung stehen und $X_3$ Wasserstoff ist oder $X_1$ Wasserstoff ist und $X_2$ und $X_3$ gemeinsam für eine zusätzliche Bindung stehen, oder in einem Salz davon die Doppelbindung zwischen den die Reste $X_1$ und $X_2$ tragenden Kohlenstoffatomen oder zwischen den die Reste $X_2$ und $X_3$ tragenden Kohlenstoffatomen zu einer Einfachbindung reduziert und gewünschtenfalls jeweils ein verfahrensgemäss erhältliches Isomerengemisch in die Komponenten auftrennt und/oder eine verfahrensgemäss erhältliche freie Verbindung I in ein pharmazeutisch verwendbares Salz überführt oder ein verfahrensgemäss erhältliches pharmazeutisch verwendbares Salz einer Verbindung I in die freie Verbindung I oder in ein anderes pharmazeutisch verwendbares Salz umwandelt und eine auf diese Weise erhaltene Verbindung der Formel

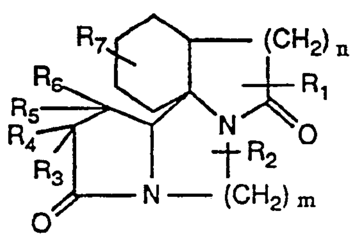

(I),

worin $R_1$, $R_2$, $R_3$, $R_5$ und $R_7$ jeweils unabhängig voneinander Wasserstoff oder Niederalkyl bedeuten, m für 2 oder 3 steht, n für 1 oder 2 steht und $R_4$ und $R_6$ entweder jeweils Wasserstoff bedeuten oder gemeinsam für eine zusätzliche Bindung stehen, in freier Form oder in pharmazeutisch verwendbarer Salzform, gegebenenfalls unter Beimischung von üblichen pharmazeutischen Hilfsstoffen, zu einem pharmazeutischen Präparat verarbeitet.

13. Verfahren zur Herstellung eines pharmazeutischen Präparats, dadurch gekennzeichnet, dass man eine Verbindung, erhältlich gemäss einem der Ansprüche 1 bis 11, in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

14. Verfahren zur Herstellung eines pharmazeutischen Präparats gemäss Anspruch 13, dadurch gekennzeichnet, dass man eine Verbindung, erhältlich gemäss einem der Ansprüche 2, 4, 6 bis 8 und 11, in freier Form oder in Form eines pharmazeutisch verwendbaren Salzes, gegebenenfalls unter Beimischung von üblichen

pharmazeutischen Hilfsstoffen, zu einem pharmazeutischen Präparat verarbeitet.

15. Verfahren gemäss einem der Ansprüche 12 bis 14 zur Herstellung eines nootrop wirksamen pharmazeutischen Präparats, dadurch gekennzeichnet, dass man einen nootrop wirksamen Wirkstoff wählt.

16. Verfahren gemäss Anspruch 14 zur Herstellung eines nootrop wirksamen pharmazeutischen Präparats, dadurch gekennzeichnet, dass man einen nootrop wirksamen Wirkstoff wählt.

17. Verwendung einer Verbindung, erhältlich gemäss einem der Ansprüche 1 bis 11, in freier Form oder in Form eines pharmazeutisch verwendbaren Salzes, zur Herstellung eines pharmazeutischen Präparats.

18. Verwendung einer Verbindung, erhältlich gemäss einem der Ansprüche 1 bis 11, in freier Form oder in Form eines pharmazeutisch verwendbaren Salzes, zur Herstellung eines pharmazeutischen Präparats auf nichtchemischem Wege.

19. Verwendung einer Verbindung gemäss Anspruch 17 oder 18 zur Herstellung eines Nootropikums.